# EUROPÄISCHE PATENTANMELDUNG

(11) **EP 3 173 107 A1**
(43) Veröffentlichungstag der Anmeldung: **31.05.2017**
(21) Anmeldenummer: 15196333.7
(22) Anmeldetag: 25.11.2015
(51) Int. Cl.: A61M 1/10

(54) **ANSCHLUSSEINRICHTUNG**

(71) Anmelder: Berlin Heart GmbH, 12247 Berlin (DE)
(72) Erfinder: MATTHES, Michael, 15345 Altlandsberg (DE); GUNDLACH, Heiko, 10435 Berlin (DE); ARSLAN, Nedim, 10717 Berlin (DE); LAUTERBACH, Gerhard, 12679 Berlin (DE); PHILLIPS, Daniel, 10249 Berlin (DE); WINTERWERBER, Kim Peter, 12357 Berlin (DE)
(74) Vertreter: Pfenning, Meinig & Partner mbB

(57) **Zusammenfassung**

Die Neuerung bezieht sich auf eine Anschlusseinrichtung (1) für den Anschluss eines rohr- oder schlauchförmigen Elementes an das Herz oder ein Blutgefäß eines Patienten mit einem Nahtring (2), der eine durch ein Verschlusselement (7) verschließbare Öffnung zur Durchführung des rohr- oder schlauchförmigen Elementes in Axialrichtung aufweist. Bei dieser ist vorgesehen, dass das Verschlusselement (7) durch wenigstens ein elastisches Befestigungselement (10) an dem Nahtring (2) befestigt ist. Es kann zudem ein radial aufweitbares Dichtelement zur Herstellung einer dichtenden Anlage des Verschlusselementes (7) an dem Nahtring (2) vorgesehen sein.

## Beschreibung

Die Erfindung liegt auf dem Gebiet des Maschinenbaus und der Mechanik und ist mit besonderem Vorteil im Bereich der Medizintechnik einsetzbar.

Konkreter bezieht sich die Erfindung auf eine Anschlusseinrichtung, mit der rohr- oder schlauchförmige Elemente an einen Hohlraum, insbesondere ein Herz oder Blutgefäß im Körper eines Patienten, angeschlossen werden können. Solche Aufgaben stellen sich beispielsweise bei einem vorübergehenden oder dauerhaften Anschluss einer Pumpe an ein Blutgefäß oder direkt an das Herz eines Patienten. Bei solchen Anwendungen wird eine Kanüle oder ein Pumpenrohr unmittelbar an einer Herzwand oder einer Gefäßwand befestigt und durch diese Wand möglichst fluiddicht durchgeführt.

Zu diesem Zweck ist es bereits bekannt, sogenannte Nahtringe zu verwenden, wobei ein Nahtring üblicherweise einen festen ringförmigen Körper, beispielsweise aus einem Metall, sowie einen mit diesem Körper verbundenen flexiblen Teil aufweist, der mittels üblicher Befestigungsmethoden an dem organischen Gewebe befestigbar ist, das die von dem Rohr oder Schlauch durchsetzte Öffnung umgibt. Beispielsweise kann diese Befestigung durch Vernähen mittels eines Fadens oder Verkleben oder Verschweißen hergestellt werden. Durch den Nahtring wird dann üblicherweise ein Rohrstutzen, ein Schlauch oder eine Kanüle gedichtet hindurchgeführt.

In einigen Fällen wird die entsprechende Öffnung im Gewebe vorübergehend oder endgültig nicht mehr benötigt, so dass es vorteilhaft ist, diese zu verschließen. Es kann in einigen Fällen hierzu vorteilhaft sein, den Nahtring am Gewebe zu belassen und die Öffnung im Nahtring zu verschließen. Dabei wird eine weitere Beschädigung von organischem Gewebe vermieden, und die Öffnung kann dicht an dem Nahtring verschlossen werden, wenn ein entsprechender Verschlussmechanismus gewählt wird. Eventuelle Unwägbarkeiten beim Verbinden oder Verschließen von organischem Gewebe treten dann nicht auf. Zudem kann der verschlossene Nahtring später auch wieder ohne großen Aufwand geöffnet werden.

Aus dem Stand der Technik ist beispielsweise im Rahmen der US-Patentschrift 7 942 805 B2 ein sogenannter Weaning-Stopfen bekannt geworden, der den Verschluss eines Nahtrings ermöglicht. Dort wird von außen mittels eines Spannrings ein Verschlusselement befestigt. ("Weaning" bezeichnet den Vorgang, bei einem Patienten nach einer Behandlung intermittierend oder endgültig auf die Unterstützung durch eine Herzpumpe zu verzichten, so dass der entsprechende Anschluss entfernt oder verschlossen werden muss.)

Der vorliegenden Erfindung liegt vor dem Hintergrund des Standes der Technik die Aufgabe zugrunde, eine Anschlusseinrichtung für den Anschluss eines rohr- oder schlauchförmigen Elementes an das Herz oder ein Blutgefäß eines Patienten zu schaffen, die mit möglichst geringem konstruktivem Aufwand in möglichst sicherer und zuverlässiger Weise den Verschluss einer Öffnung zulässt, wobei zudem der Aufwand beim Verschließen der Öffnung gering gehalten und der Patient möglichst weitgehend geschont werden soll.

Die Aufgabe wird mit den Merkmalen der Neuerung gemäß Patentanspruch 1 gelöst. Weitere Ausgestaltungen der Neuerung sind den Unteransprüchen 2 bis 15 zu entnehmen.

Die Neuerung bezieht sich mithin auf eine Anschlusseinrichtung für den Anschluss eines rohr- oder schlauchförmigen Elementes an das Herz oder ein Blutgefäß eines Patienten mit einem Nahtring, der eine durch ein Verschlusselement verschließbare Öffnung zur Durchführung des rohr- oder schlauchförmigen Elementes in Axialrichtung aufweist. Die Aufgabe wird gemäß der Neuerung dadurch gelöst, dass das Verschlusselement durch wenigstens ein elastisches Befestigungselement an dem Nahtring befestigt ist.

Durch das elastische Befestigungselement kann einerseits mittels einer Klemmeinrichtung ohne größeren Aufwand ein Verschlusselement an einem Nahtring befestigt werden, und andererseits kann das Befestigungselement potenziell wegen seiner Elastizität wenigstens teilweise auch als Dichtelement dienen. Hierdurch ist eine Befestigung des Verschlusselementes am Nahtring möglich, ohne dass der Nahtring in größerem Maß bewegt oder manipuliert werden muss. Die Befestigung durch das elastische Befestigungselement funktioniert dauerhaft und garantiert den mechanischen Halt des Verschlusselementes unabhängig von Bewegungen des Patienten und Änderung der Umgebungsbedingungen. Insbesondere ist eine Verbindung mittels eines elastischen Befestigungselementes in einfacher Weise und ohne größere Belastung des Patienten mit einem minimalen Eingriff in den Körper des Patienten herzustellen. Das Verschlusselement kann an einer Stirnfläche des Nahtrings oder an einer insbesondere zylindrischen Innenfläche dichtend anliegen. Es ist generell auch denkbar, dass das Verschlusselement an einer äußeren Umfangsfläche des Nahtrings dichtend anliegt.

Eine Ausgestaltung der Neuerung sieht vor, dass das Verschlusselement ein an seinem äußeren Umfang an dem Nahtring dichtendes Dichtelement sowie eine Einrichtung zur Aufweitung des Dichtelementes in Radialrichtung des Nahtrings aufweist. Das Dichtelement ist üblicherweise verformbar, insbesondere elastisch, und kann beispielsweise aus einem Elastomer bestehen oder ein Elastomer aufweisen. Es kann beispielsweise auch aus einem plastisch verformbaren Stoff mit Klebeigenschaften, typischerweise einem Kleber, bestehen oder mittels eines Klebers mit dem Nahtring verbunden sein. Es kann beispielsweise an einer zylindrischen oder anders geformten Innenfläche des Nahtrings oder an anderen Flächen des Nahtrings, beispielsweise an einer Stirnfläche, einer Schräge oder in einer Nut des Nahtrings, durch dichte Anlage dichten. Das Dichtelement kann beispielsweise mit dem Befestigungselement identisch oder Teil des Befestigungselementes sein; es kann jedoch auch unabhängig vom Befestigungselement vorgesehen sein.

Das Dichtelement kann massiv oder hohl ausgebildet sein, beispielsweise als Hohlkörper mit einem zentralen Hohlraum oder auch als geschlossen- oder offenporiger Schaumstoff, oder es kann einen solchen Schaumstoff aufweisen. Möglich ist (dies gilt für alle Ausführungsformen der vorliegenden Schutzrechtsanmeldung) statt eines Schaumstoffs auch offenporiges Textilgewebe zum Fördern des Anwachsens von organischem, insbesondere menschlichem Gewebe. Dies kann beispielsweise Graft- und/oder Velours-Material umfassen.

Das Dichtelement, das Befestigungselement und das Verschlusselement können jeweils ganz oder teilweise aus einem Metall oder einem Kunststoff bestehen, ebenso aus einem aufgeschäumten Material, beispielsweise auch Metallschaum aus einer offenporigen Metallstruktur. Ein in Frage kommendes Metall ist dabei Titan. Die Oberflächen oder Oberflächenbereiche können (dies gilt für alle in diesem Absatz genannten Elemente) dabei derart texturiert sein, dass das Einwachsen begünstigt wird. Auch Textilgewebe oder sonstiges Gewebe kann mit oder ohne Texturierung verwendet werden.

Allgemein kommen für die genannten Bauteile biokompatible Materialien in Frage, die einwachsfähig sind.

Eine weitere Lösung im Rahmen der vorliegenden Neuerung kann vorsehen, dass das Dichtelement in Axialrichtung komprimierbar und dadurch in radialer Richtung aufweitbar ist. Diese Lösung kann beispielsweise vorsehen, dass das Dichtelement aus einem nicht volumenkompressiblen Elastomer besteht und beispielsweise ringförmig ausgebildet ist, oder auch, dass das Dichtelement ein hohles Elastomerelement ist oder umfasst, das unter Komprimierung eines im Hohlraum befindlichen Mediums verformt werden kann.

Weiter kann im Rahmen der Neuerung vorgesehen sein, dass das Verschlusselement eine an seinem Umfang umlaufende Ausnehmung für das in Axialrichtung komprimierbare Dichtelement aufweist. Es kann beispielsweise das Verschlusselement ein scheibenförmiges oder topfförmiges Element aufweisen, das an seinem Umfang eine entsprechende Ausnehmung aufweist, die beispielsweise als umlaufende Nut ausgebildet sein kann.

Die Außenkontur des Verschlusselements kann dabei rund, insbesondere kreisrund, sein, sie kann jedoch auch elliptisch oder viereckig oder mehreckig (polygon), jeweils beispielsweise unter Abrundung der Ecken, gestaltet sein. Der Begriff "radial" bedeutet in diesem Fall z. B. vom Flächenschwerpunkt oder Flächenmittelpunkt nach außen zum Umfang gerichtet.

Es kann auch als implementierbare Lösung vorgesehen sein, dass das Verschlusselement wenigstens zwei gesonderte und in Axialrichtung gegeneinanderpressbare Elemente aufweist, zwischen denen ein radial nach außen offener Zwischenraum, insbesondere eine am Umfang des Verschlusselements umlaufende Nut, zur Aufnahme des Dichtelementes gebildet ist. Das Verschlusselement kann hierzu zwei in Axialrichtung, d. h. in Durchgangsrichtung der Anschlusseinrichtung, hintereinander liegende und gegeneinander in Axialrichtung bewegliche Elemente aufweisen, zwischen denen ein massives oder hohles Elastomerelement als Dichtelement angeordnet sein kann. Die gegeneinanderpressbaren Elemente können beispielsweise als flache Scheiben oder kalottenartige Körper ausgebildet sein, oder es kann einer der Körper als Scheibe und der andere Körper als gegen die Scheibe pressbarer Stempel ausgebildet sein. Das Dichtelement kann auf ganzer Breite zwischen den beiden gegeneinanderpressbaren Elementen liegen oder als ringförmiger Körper ausgebildet sein.

Bei einer axialen Pressung wird das Dichtelement radial nach außen expandiert, bis es gegen eine Dichtfläche des Nahtrings stößt und dort dichtet. In einer besonderen Ausbildung der Neuerung kann am Umfang des Verschlusselementes eine umlaufende Nut zur Aufnahme des Dichtelementes gebildet sein. Die Nut kann dann derart verformbar sein, dass die Breite der Nut in Axialrichtung verringerbar ist, so dass das Dichtelement aus der Nut heraus und in radialer Richtung nach außen gegen den Nahtring drückbar ist. Dies kann beispielsweise dadurch realisiert sein, dass die verschiedenen Seitenwände der Nut durch die verschiedenen gegeneinanderpressbaren Elemente gebildet sind.

Insbesondere für den Fall, dass an dem Verschlusselement eine radial außen umlaufende Nut angeordnet ist, kann weiterhin vorgesehen sein, dass am Boden der Nut wenigstens an einem der beiden gegeneinanderpressbaren Elemente eine umlaufende und radial nach außen weisende Schräge gebildet ist und dass insbesondere der Boden der Nut im Querschnitt V-förmig ausgebildet ist, wobei jeder der beiden Schenkel des V-förmigen Bodens von einem der Elemente gebildet ist. Eine derartige Ausformung der Nut fördert speziell die Umsetzung einer Kompression des Dichtelementes in Axialrichtung in eine Expansion in Radialrichtung nach außen.

Die beiden gegeneinanderpressbaren Elemente können durch verschiedene Mechanismen axial gegeneinandergedrückt werden, beispielsweise dadurch, dass eines der Elemente an einem Anschlag des Nahtrings anliegt und das zweite Element durch Klemmelemente gegen das erste Element gepresst wird, die an den Nahtring abgestützt sind. Soll eine Kompression der beiden gegeneinanderpressbaren Elemente möglichst einfach und ohne eine Bewegung des Nahtrings bzw. ohne eine Belastung des Nahtrings mit Kräften geschehen, so kann beispielsweise vorgesehen sein, dass die beiden gegeneinanderpressbaren Elemente durch eine zentrale Schraubverbindung miteinander verbunden sind. Damit müssen nur die beiden gegeneinanderpressbaren Elemente miteinander oder mit einem dritten Element verschraubt werden, wobei Kräfte auf den Nahtring entweder gar nicht auftreten oder in engen Grenzen gehalten werden können.

Konkret kann hierzu beispielsweise vorgesehen sein, dass wenigstens ein Gewindestift eine Öffnung eines der gegeneinanderpressbaren Elemente ohne Gewindeeingriff durchsetzt und dass wenigstens eine Gewindemutter auf den Gewindestift zum Gegeneinanderpressen der Elemente des Verschlusselements schraubbar ist. Eine derartige Schraubverbindung ist einerseits zum Komprimieren des Dichtelementes leicht spannbar und andererseits ebenso leicht wieder lösbar, um das Verschlusselement beispielsweise wieder zu entfernen. Außerdem ist durch die Schraubverbindung die auf das Dichtelement wirkende Kraft stufenlos einstellbar. Das Gewinde der Schraubverbindung sollte eine derart flache Steigung haben, dass die Schraubverbindung selbsthemmend ist.

Es kann im Rahmen der Neuerung auch vorgesehen sein, dass die beiden gegeneinanderpressbaren Elemente ineinandergreifende zentrische Gewinde aufweisen und gegeneinander verdrehbar sind. In diesem Fall kann eines der beiden gegeneinanderpressbaren Elemente ein Außengewinde und das andere Element ein Innengewinde aufweisen, die unmittelbar miteinander verschraubt sind. Diese Gewinde können entweder im Bereich des äußeren Umfangs des Verschlusselements oder auch radial weiter innen an entsprechend vorgesehenen Stutzen bzw. rohrförmigen Elementen vorgesehen sein,

Allgemein kann auch für den Fall, dass kein Gewinde zwischen den gegeneinanderpressbaren Elementen vorhanden ist, vorgesehen sein, dass die beiden gegeneinanderpressbaren Elemente eine Führungseinrichtung zur Führung ihrer Relativbewegung in Axialrichtung aufweisen. Dies ist insbesondere dann wichtig, wenn der axiale Pressdruck durch Klemmelemente realisiert wird, die selbst keine Führung der beiden gegeneinanderpressbaren Elemente in Axialrichtung bewirken.

Jedenfalls kann auch vorgesehen sein, dass an dem Verschlusselement ein Anschlag zur Begrenzung der Relativbewegung der beiden gegeneinanderpressbaren Elemente in Axialrichtung vorgesehen ist. Hierdurch wird die Kompression des Dichtelementes in axialer Richtung begrenzt, so dass beispielsweise das Risiko einer Zerstörung des Dichtelementes oder eines vollständigen Herausdrückens des Dichtelementes aus dem Zwischenraum zwischen den gegeneinanderpressbaren Elementen begrenzt ist.

Eine vorteilhafte Ausgestaltung der Neuerung kann beispielsweise vorsehen, dass das Dichtelement eine Elastomerdichtung mit insbesondere kreisrundem, ovalem, dreieckigem oder viereckigem Querschnitt ist. Die Elastomerdichtung kann beispielsweise aus einem Gummi, einem Silikonelastomer, einem offenporigen Textilgewebe (z. B. einem Velours- oder Graftmaterial) oder einem anderen vergleichbaren Stoff bestehen und beispielsweise auch als geschlossen- oder offenporiger Schaumstoff oder als texturiertes Material ausgestaltet sein. Weiter ist es auch möglich, dass die Elastomerdichtung einen oder mehrere Hohlräume aufweist, die mit einem Fluid, einem Gel oder einem Gas gefüllt sind, wobei der Hohlraum geschlossen und verformbar sein kann. Es kann auch das Einfüllen eines Mediums in den Hohlraum zur Verformung des Dichtelementes vorgesehen sein.

Hierzu kann beispielsweise an dem Dichtelement eine Fülleinrichtung vorgesehen sein, die öffenbar und verschließbar ist. Es kann beispielsweise in den Hohlraum eines derartigen Dichtelementes auch ein Stoff eingefüllt werden, der zunächst fließfähig ist und dessen Einfüllen zu einer radialen Expansion des Dichtelementes führt, wobei der Stoff später aushärtbar oder versteifbar ist, um das Dichtelement im expandierten Zustand zu verfestigen. Ein solcher Stoff kann beispielsweise auch mittels einer Injektionsnadel ohne eine gesonderte Fülleinrichtung in den Hohlraum eines Dichtelementes eingespritzt werden.

In diesem Zusammenhang soll festgehalten werden, dass eine mögliche Ausgestaltung der Neuerung vorsehen kann, dass das Dichtelement ein verformbarer, insbesondere elastischer und mit einem Gas, einer Flüssigkeit oder einem Gel füllbarer Hohlkörper, insbesondere in Torus- oder Kugelform, ist.

Grundsätzlich kann die Anschlusseinrichtung auch derart gestaltet sein, dass das Verschlusselement einen von einer elastischen Wand umgebenen Hohlraum aufweist, der zusammen mit der elastischen Wand durch Einschieben eines zapfenartigen Körpers oder durch Spreizen eines Spreizelementes innerhalb des Hohlraums radial aufweitbar ist. Dazu kann beispielsweise das Verschlusselement ein Dichtelement in Form eines Torus oder eines Dichtungsrings aufweisen, in dessen axialer Durchgangsöffnung ein zapfenförmig oder konisch sich erweiternder Körper eingeschoben wird, der den torusförmigen Körper in Bezug auf seine Symmetrieachse radial aufweitet.

Es kann jedoch auch ein Spreizelement innerhalb des Hohlraums eines Dichtelementes vorgesehen sein, das von außen betätigbar ist, so dass die den Hohlraum begrenzenden Wände des Dichtelementes radial nach außen erweitert werden, bis sie an einer Dichtfläche des Nahtrings dichten.

Im Folgenden wird die Neuerung anhand von Ausführungsbeispielen in Figuren einer Zeichnung gezeigt und nachfolgend erläutert. Dabei zeigt
- Fig. 1: in einem schematischen Schnitt eine Anschlusseinrichtung mit einem Dichtungsring, der als Befestigungselement und als Dichtelement dient,
- Fig. 2: eine Anschlusseinrichtung mit einem hohlen Dichtungsring,
- Fig. 3: eine Anschlusseinrichtung mit einem elastischen Deckel,
- Fig. 4: eine Anschlusseinrichtung mit einem Deckel, der elastische Haken aufweist,
- Fig. 5a: in einem Halbschnitt eine Anschlusseinrichtung mit einem Deckel und einer Überwurfmutter,
- Fig. 5b: einen Federring als Detail aus Figur 5a,
- Fig. 6: im Schnitt eine Anschlusseinrichtung mit einem hohlen Gummikörper,
- Fig. 7: eine Anschlusseinrichtung mit einem hohlen verdrehbaren Gummikörper,
- Fig. 8: eine Anschlusseinrichtung mit einem hohlen Dichtelement und einer Spreizvorrichtung,
- Fig. 9: eine Anschlusseinrichtung mit einem Ventil, auf der rechten Seite mit einem eingefügten Pumpenrohr,
- Fig. 10: eine Anschlusseinrichtung mit einem hohlen Dichtungsring und einem in diesen einführbaren Zapfen zur Aufweitung,
- Fig. 11: eine Anschlusseinrichtung mit einem konischen Stopfen, der unmittelbar in die Öffnung des Nahtrings gedrückt werden kann, sowie
- Fig. 12: eine Anschlusseinrichtung mit einem Stopfen, der radial außen auf eine Außenfläche des Nahtrings aufgeklemmt ist.

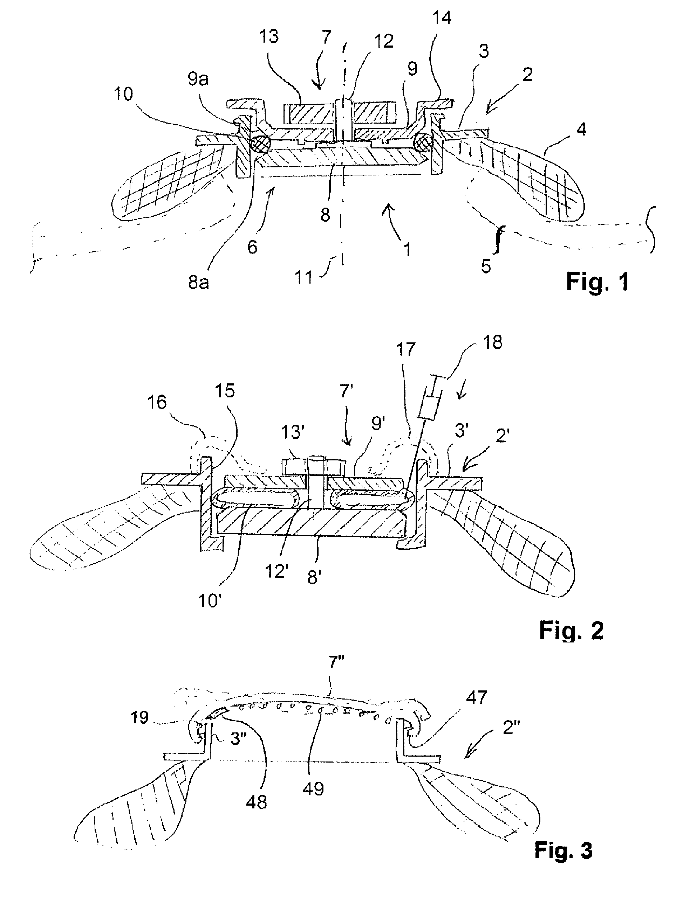

Figur 1 zeigt in einem Schnitt schematisch eine Anschlusseinrichtung 1 für den Anschluss eines rohr- oder schlauchförmigen Elementes an das Herz eines Patienten. Die Anschlusseinrichtung 1 weist einen Nahtring 2 auf, der seinerseits einen harten Teil 3, ausgebildet als Metallring, und einen eher weichen Bereich 4 aufweist, der mit organischem Gewebe 5, beispielsweise eines Patientenherzens, mittels Nadel und Faden vernähbar ist. Der Bereich 4 kann beispielsweise als textiles Gewebe ausgebildet sein.

Der Nahtring 2 weist eine zentrische zylindrische Öffnung 6 auf, durch die im Betrieb einer Herzpumpe ein Rohr, eine Kanüle oder ein Schlauch hindurchgeführt und dort üblicherweise gedichtet ist. In der Darstellung der Figur 1 ist diese Öffnung 6 mittels eines Verschlusselementes 7 dicht verschlossen. Das Verschlusselement 7 weist zwei gegeneinanderpressbare Elemente 8, 9 auf, die jeweils zylindersymmetrisch ausgebildet und im radial inneren Teil scheibenartig ausgebildet sind. Die beiden Elemente 8, 9 weisen im radial äußeren Bereich Schrägen 8a, 9a auf, die einander zugewandt sind, am Umfang des Nahtrings umlaufen und gemeinsam eine Nut 8a, 9a mit V-förmigen Seitenwänden bilden, in der ein Dichtring 10 liegt. Werden die beiden Elemente 8, 9 in Axialrichtung 11, die der Durchlassrichtung der Öffnung 6 und der Förderrichtung einer Flüssigkeit bei offenem Anschluss entspricht, gegeneinandergepresst, so wird der Dichtring 10 in axialer Richtung komprimiert und damit in radialer Richtung gegen die Innenwand des metallischen Teils 3 des Nahtrings 2 gedrückt. Damit ist das Verschlusselement 7 dicht in dem Nahtring 2 verklemmt.

Um ein axiales Zusammenpressen der Elemente 8, 9 zu gewährleisten, ist mit dem Element 8 eine Gewindestange 12 verbunden, die durch eine zentrische Öffnung in dem Element 9 hindurchragt und auf die eine Gewindemutter 13 aufschraubbar ist. Das Element 9 weist eine Krempe 14 auf, die über den Rand des metallischen Teils 3 des Nahtrings 2 hinüberragt. Beim Manipulieren des Verschlusselementes 7 kann beispielsweise ein Operateur an der Krempe 14 das Verschlusselement fassen und die Gewindemutter 13 drehen, um die Elemente 8, 9 gegeneinanderzupressen oder beim Lösen der Mutter 13 voneinander zu lösen. Den eigentlichen Nahtring 2 muss der Operateur beim Anbringen und Lösen des Verschlusselementes 7 nicht notwendigerweise anfassen, und die auf den Nahtring 2 wirkenden Kräfte während des Verschluss- oder Lösevorgangs sind begrenzt. Damit wird das Risiko, dass der weiche Teil 4 des Nahtrings 2 sich von dem organischen Gewebe 5 der Herzwand löst, minimiert.

Der Dichtring 10 kann beispielsweise aus Gummi oder einem Silikonelastomer bestehen und im Querschnitt fast beliebige Formen annehmen, die nur zur Form der Nut 8a, 9a passen müssen, die zwischen den Elementen 8, 9 gebildet ist. Günstige Querschnitte können beispielsweise dreieckig, kreisrund oder elliptisch sein.

Figur 2 zeigt einen Nahtring 2', in den ein Verschlusselement 7' eingesetzt ist. Das Verschlusselement 7' weist zwei axial zusammenpressbare Elemente 8', 9' auf, die wie in Figur 1 mittels einer Kombination aus einer Gewindestange 12' und einer Gewindemutter 13' zusammenpressbar und lösbar sind. Als Dichtelement ist ein ringförmiges, hohles Elastomerelement 10' vorgesehen, das bei Kompression der Elemente 8', 9' radial nach außen expandiert und an der Innenwand 15 des metallischen Teils 3' des Nahtrings 2' dichtet.

Alternativ sind schematisch Federklemmen 16, 17 gestrichelt angedeutet, die zusätzlich zu dem oder anstelle des Kompressionsmechanismus, der aus der Gewindestange 12' und der Mutter 13' besteht, vorgesehen sein können, um die Elemente 8', 9' axial zu komprimieren.

Zudem ist in Figur 2 noch ein anderer Mechanismus angedeutet, der zu einer Dichtung des Dichtelementes 10' an der Innenwand 15 des Nahtrings 2' führt, nämlich das Einbringen einer Substanz, beispielsweise eines Gases, eines Gels oder einer Flüssigkeit, in den Hohlraum des Dichtelementes 10' mittels eines Spritzenmechanismus 18, der im einfachsten Fall durch eine Injektionsspritze verkörpert sein kann. Zum Dichten des Verschlusselementes 7' an dem Nahtring 2' kann dann mittels einer Spritze Gas oder ein Gel oder eine Flüssigkeit über eine eingestochene Nadel in das Dichtelement 10' eingebracht werden, um das Dichtelement zu expandieren. Aufgrund fehlender Expansionsmöglichkeiten in axialer Richtung wird das Dichtelement 10' radial nach außen expandieren und an dem Nahtring 2' dichten. Zusätzlich kann in einem Verfahrensschritt vorgesehen sein, dass die Substanz im Hohlraum des Dichtelements 10' verfestigt wird, beispielsweise durch Vernetzung infolge thermischer Behandlung oder Bestrahlung, und dass damit das Verschlusselement in dem Nahtring 2' fixiert wird.

Figur 3 zeigt einen Verschlussmechanismus mit einem Verschlusselement 7" in Form eines elastischen Deckels, der auf den festen Bereich 3" des Nahtrings 2" aufschnappbar ist. Das Verschlusselement 7" kann beispielsweise derart geformt sein, dass es zwei stabile Formzustände hat, zwischen denen es bewegt werden kann, wobei in einem stabilen Formzustand das Verschlusselement über den Rand des metallischen Teils 3" und einen radial an diesem umlaufenden Steg 19 aufschnappbar ist. Das Verschlusselement 7" weist zum Verhaken unter dem Steg 19 eine nach innen gebogene umlaufende Krempe 47 auf. Der runde Deckel 7" bildet für sich das Verschlusselement sowie das elastische Befestigungselement und auch das Dichtelement.

Der elastische Deckel 7" kann aus einem texturierten Material, wie beispielsweise einem oberflächentexturierten Metall, z. B. Titan, einem Metallschaum, einem Textil oder Gewebe, bestehen, das das Einwachsen begünstigt. Dabei ist auch denkbar, dass der Deckel erst durch das Einwachsen dicht wird. Die Texturierung oder Abdeckung mit einem Textil oder Gewebe ist in Figur 2 mit 49 bezeichnet. Zusätzlich kann der Deckel auf seiner dem Nahtring zugewandten Seite einen Dichtungsring 48 aus einem Elastomer oder beispielsweise auch mit einer texturierten Oberfläche aufweisen.

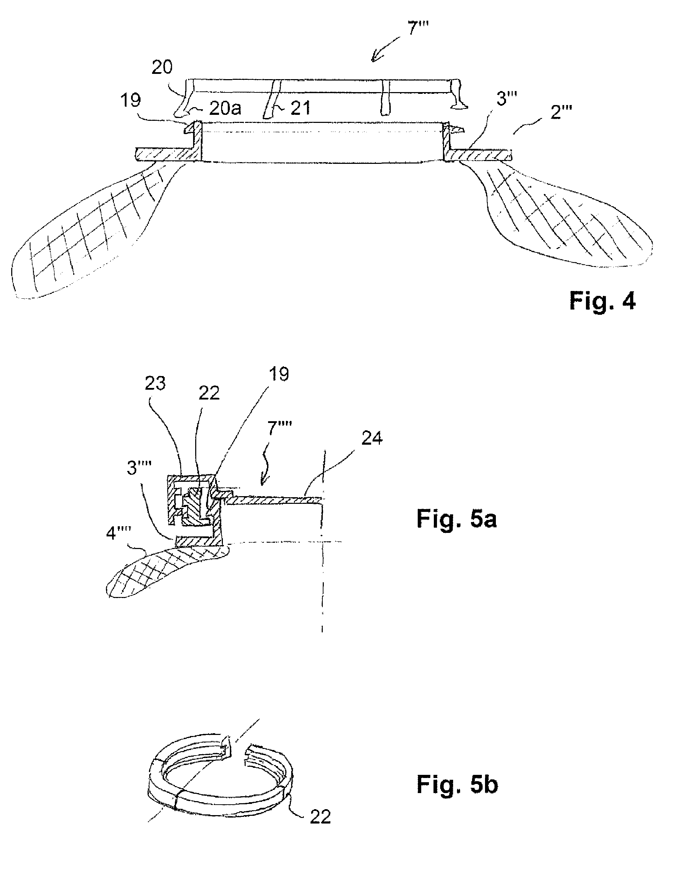

Figur 4 zeigt im Schnitt eine Anschlusseinrichtung mit einem Nahtring 2"' und einem Verschlusselement 7"', wobei der metallische Teil 3'" des Nahtrings einen außen umlaufenden Steg 19 ähnlich dem Steg 19 der Figur 3 aufweist, wobei jedoch im Ausführungsbeispiel der Figur 4 nicht eine umlaufende Krempe 47 verformt und an dem umlaufenden Steg 19 verhakt wird, sondern lediglich einzelne elastische Haken 20, 21, die jeweils radial an ihrem freien Ende auslenkbar sind und dort einen Haken 20a tragen. Das Verschlusselement 7'" stellt dabei selbst das Dichtelement dar, das an dem metallischen Teil 3"' des Nahtrings dichtet. Die Befestigungselemente sind in diesem Beispiel durch die Elemente 20, 20a, 21 gebildet. Im Besonderen ist es möglich, eine Langzeitdichtwirkung zu erzielen, indem man mit einer Textur 49' aus Metall oder Textil an den Innenflächen des Verschlusselementes das Anwachsen von Gewebe fördert. Eine zusätzliche Dichtung wird somit durch Gewebe erzielt.

Figur 5a zeigt einen Halbschnitt eines Nahtrings mit einem weichen Bereich 4"" sowie einem metallischen Bereich 3"", auf den ein Verschlusselement 7"" aufgesetzt ist. Das Verschlusselement 7"" weist einen Federring 22 auf, der um einen rohrstutzenförmigen Teil des metallischen Teils 3"" herumlegbar und dort hinter einem Steg 19 verhakbar ist. Auf dem Federring 22, der für sich in Figur 5b in perspektivischer Ansicht dargestellt und für die elastische Aufweitbarkeit geschlitzt ist, ist eine Überwurfmutter 23 aufbringbar, die mit einem runden Deckel 24 des Verschlusselementes 7"" verbindbar oder fest verbunden ist. Zum Verschließen der Anschlusseinrichtung wird somit über den metallischen Teil 3"" des Nahtrings zunächst der Federring 22 elastisch gelegt bzw. aufgeklemmt und darauf die Überwurfmutter 23 mit dem Deckel 24 geschraubt.

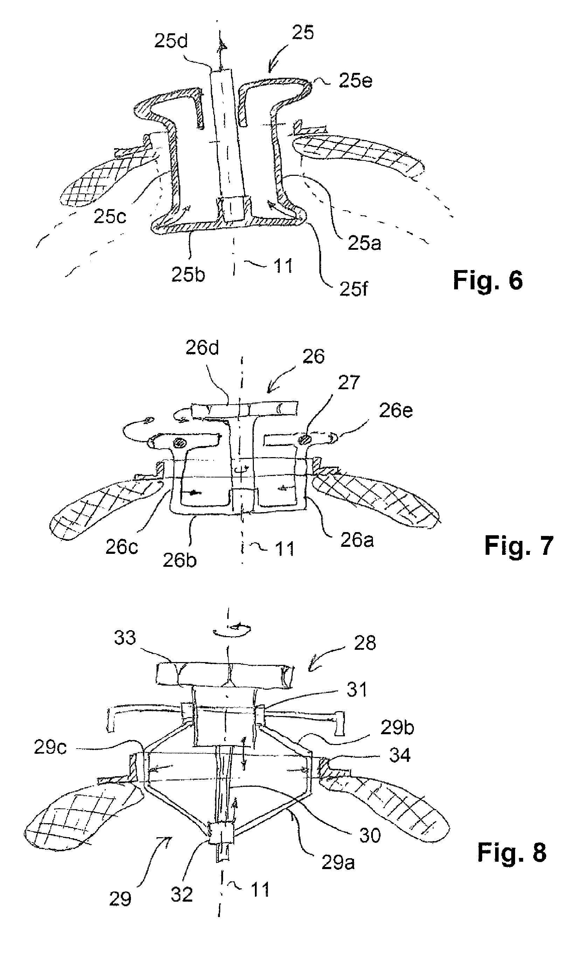

Die in den Figuren 6 und 7 dargestellten Verschlusselemente 25, 26 weisen jeweils einen aus einem Elastomer bestehenden topfartigen Körper 25a, 26a mit einem Boden 25b, 26b und einem zylindrischen Wandteil 25c, 26c sowie eine jeweils mit dem Boden 25b, 26b fest verbundene Stütze 25d, 26d auf. Zum Einbringen und zum Lösen der Verschlusselemente 25, 26 in den Öffnungen der jeweiligen Nahtringe in den Figuren 6 und 7 kann jeweils die Stütze 25d, 26d gegenüber dem topfartigen Körper 25a, 26a um die Achse 11 gedreht werden. In der Folge dieser Relativbewegung zwischen den Stützen und den topfartigen Körpern fällt jeweils der topfartige Körper 25a, 26a radial zusammen und löst sich von der Dichtfläche an dem jeweiligen Nahtring. Wird die Relativdrehung zwischen den Stützen und den topfartigen Körpern zurückgenommen, so weitet sich der jeweilige topfartige Körper 25a, 26a radial auf, bis er an einer zylindrischen Dichtfläche des jeweiligen Nahtrings wieder dichtend anliegt.

Die topfartigen Körper 25a, 26a weisen zur Fixierung an dem jeweiligen Nahtring eine oder mehrere radiale Erweiterungen 25e, 25f bzw. 26e auf, die auf einer oder beiden Seiten des Nahtrings in Axialrichtung jeweils Anschläge bilden und damit ein Herausrutschen der Verschlusselemente 25, 26 aus der Öffnung des jeweiligen Nahtrings in Axialrichtung verhindern.

Die Stützen 25d, 26d können beispielsweise Verdickungen wie in Figur 7 dargestellt aufweisen, um ein besseres Greifen zur Verdrehung zu erlauben.

Innerhalb der topfartigen Körper 25a, 26a können, eingelassen in eine Wand des jeweiligen Körpers, federnde Stützelemente in Form von Drahtringen eingelassen sein, die bei Wegfallen einer Torsionskraft elastisch auf die Wiederherstellung des expandierten Zustands hinwirken. In Figur 7 ist beispielhaft ein derartiger Körper in Form eines runden Drahtrings 27 gezeigt.

Figur 8 zeigt ein Verschlusselement 28, das ein hohles Dichtelement 29, beispielsweise in Form eines ballonartigen Körpers aus einem Elastomer, aufweist, wobei tellerartige Bereiche 29a, 29b einander symmetrisch gegenüberliegen und durch einen zylindrischen Wandbereich 29c miteinander verbunden sind. Die tellerartigen Bereiche 29a, 29b weisen eine Steifigkeit auf, die bei einer axialen Annäherung aneinander entlang der Achse 11 ein Abflachen der tellerartigen Bereiche unter gleichzeitiger radialer Aufweitung zur Folge hat. Es ist gemäß Figur 8 eine Spindel 30 vorgesehen, die in einer Führung 31 geführt ist, welche mit dem oberen tellerförmigen Bereich 29b zusammenhängt. Zentrisch in dem tellerartigen Bereich 29a ist eine Spindelmutter 32 befestigt, die mit der Spindel 30 zusammenwirkt. Wird die Spindel 30 mittels des mit ihr fest verbundenen Kopfes 33 gedreht, so wird je nach Drehrichtung die Entfernung zwischen der Spindelmutter 32 und dem Kopf 33 verringert oder vergrößert und infolgedessen das Dichtelement 29 radial aufgeweitet oder komprimiert. Somit kann bei radialer Aufweitung des Dichtelements 29 der Wandbereich 29c an einer Dichtfläche des Nahtrings 34 dichten.

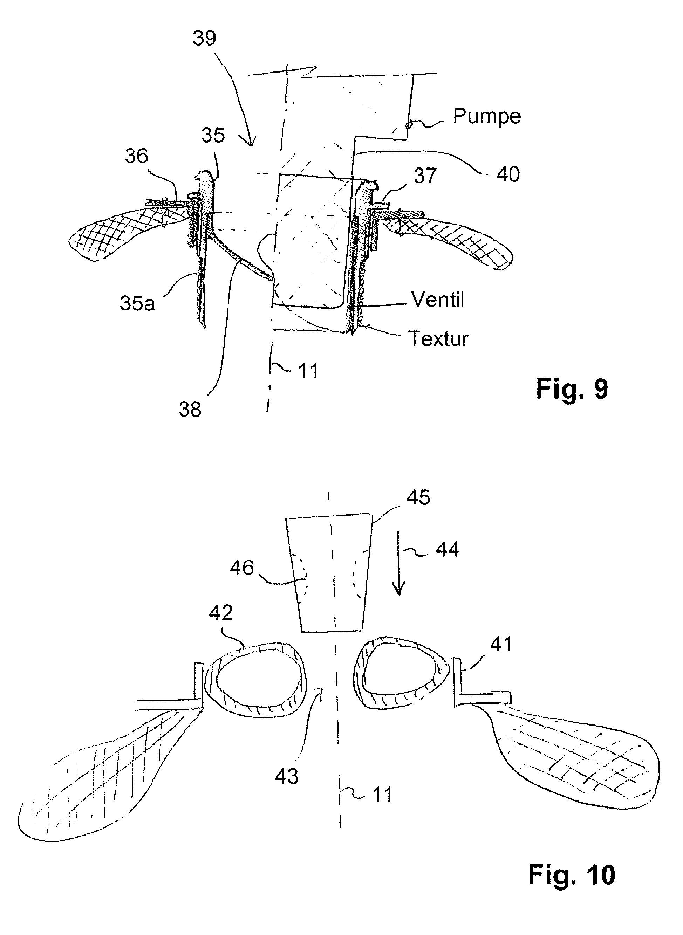

Figur 9 zeigt in einer Schnittdarstellung ein Verschlusselement 35, das einen Rohrstutzen 35a aufweist, der durch einen Nahtring 36 hindurchgeschoben wird, bis ein Flansch 37 auf dem Nahtring aufliegt. Der Rohrstutzen 35a weist an seiner Innenseite einen oder mehrere auslenkbare elastische Schwenkelemente 38 auf, die beispielsweise jeweils Segmente einer kreisförmigen Scheibe bilden können und die im entspannten Zustand die Öffnung 39 des Rohrstutzens 35a nach Art eines Ventils verschließen.

Soll ein Pumpenrohr 40 oder ein schlauchartiger oder kanülenartiger Gegenstand durch die Öffnung 39 hindurchgeschoben werden, so weichen die Schwenkelemente 38 zurück und machen für den eingeschobenen Gegenstand Platz, wie im rechten Teil der Darstellung der Figur 9 rechts von der Mittelachse 11 dargestellt ist.

Teile des Rohrstutzens 35a, insbesondere an seiner Außenseite, können eine texturierte Metalloberfläche, insbesondere eine texturierte Titanoberfläche aufweisen, die das Zellwachstum nach einer Implantation in einen lebenden Organismus fördert und damit das Einwachsen und auch die Abdichtung durch lebendes Gewebe beschleunigt. Eine solche Texturierung kann auch an den bereits oben beschriebenen festen, insbesondere metallischen Teilen der Verschlusselemente vorgesehen sein, um jeweils nach einem Einbringen in einen Patientenkörper das Einwachsen zu erleichtern bzw. zu beschleunigen.

Figur 10 zeigt innerhalb eines Nahtrings 41 ein hohles Dichtelement 42 in Form eines hohlen Elastomertorus, in dessen axiale Öffnung 43 entlang der Achse 11 in Richtung des Pfeils 44 ein konischer Stopfen 45 einschiebbar ist. Der Stopfen 45 wird in der Öffnung 43 elastisch verklemmt und gehalten und kann zur besseren Fixierung eine in Figur 10 gestrichelt dargestellte Kehlung 46 aufweisen. Durch das Einschieben des Stopfens 45 in den Dichtkörper 42 wird dieser radial aufgeweitet und dichtet an der zylindrischen Innenfläche des Nahtrings 41.

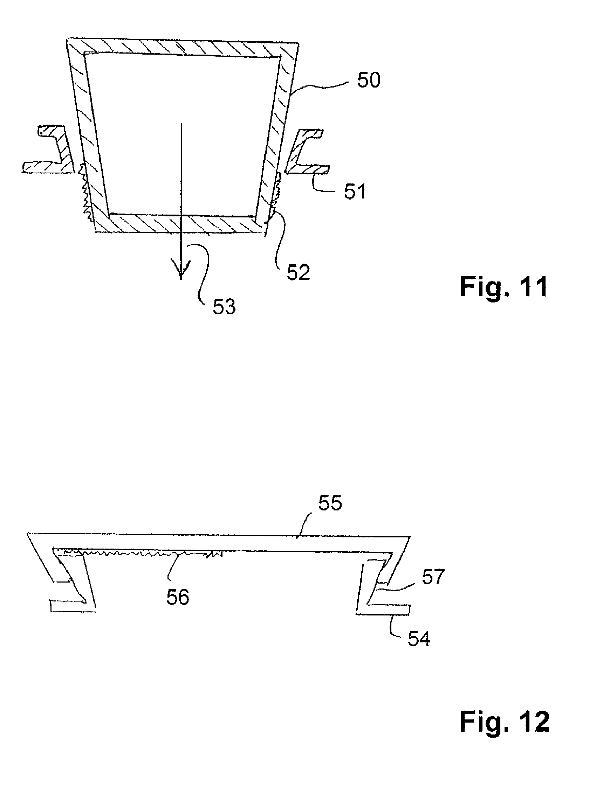

Figur 11 zeigt einen Nahtring 51, der selbst eine konische Innenkontur aufweist, jedoch auch eine zylindrische Innenkontur haben kann. Ein konischer Stopfen 50 kann hohl ausgebildet sein und aus einem Kunststoff, Elastomer oder einem Metall, auch einem geschäumten Metall, einer offenporigen Metallstruktur und insbesondere Titan bestehen. Der Stopfen kann gasgefüllt sein und wird in Richtung des Pfeils 53 axial in die Öffnung des Nahtrings gedrückt. Der Stopfen kann an seinem Umfang wenigstens teilweise, insbesondere im Dichtbereich, an seiner Oberfläche texturiert oder mit einem texturierten Stoff beschichtet sein, um das Einwachsen zu fördern (siehe Bezugszeichen 52).

Figur 12 zeigt einen Nahtring 54, der eine konische Außenkontur 57 aufweist, auf die eine umlaufende Krempe des elastischen Verschlusselementes 55 aufgeklemmt ist, um entweder dort im Bereich der Mantelfläche des Nahtrings oder an dessen Stirnseite zu dichten. Das Verschlusselement 55 kann ganz oder teilweise mit einer Texturierung 56 versehen sein oder beispielsweise aus einer offenporigen Metallstruktur bzw. einem Metallschaum oder Metallgewebe bestehen.

Die in den Ansprüchen definierten und anhand der Ausführungsbeispiele dargestellten Realisierungen der Neuerung erlauben ein Verschließen der Öffnung eines Nahtrings unter Vermeidung zu starker Bewegungen oder Krafteinwirkungen auf den Nahtring, so dass ein Verschluss durch ein Verschlusselement jeweils ohne Gefährdung des Patienten und mit minimaler Einwirkung ermöglicht ist.

## Patentansprüche

1. Anschlusseinrichtung (1) für den Anschluss eines rohr- oder schlauchförmigen Elementes an das Herz oder ein Blutgefäß eines Patienten mit einem Nahtring (2, 2', 2", 2'", 34, 36, 37, 41), der eine durch ein Verschlusselement (7, 7', 7", 7"', 7"", 25, 26, 28, 35, 39, 45) verschließbare Öffnung zur Durchführung des rohr- oder schlauchförmigen Elementes in Axialrichtung (11) aufweist, **dadurch gekennzeichnet, dass** das Verschlusselement durch wenigstens ein elastisches Befestigungselement (7", 10, 10', 20, 20a, 22, 25a, 26a, 29a, 38, 42) an dem Nahtring befestigt ist.

2. Anschlusseinrichtung nach Anspruch 1, **dadurch gekennzeichnet, dass** das Verschlusselement (7, 7', 7", 7'", 7"", 25, 26, 28, 35, 39, 45) ein an seinem äußeren Umfang an dem Nahtring (2, 2', 2", 2"', 34, 36, 37, 41) dichtendes Dichtelement (10, 10', 20, 22, 25a, 26a, 29a, 42) sowie eine Einrichtung zur Aufweitung des Dichtelementes in Radialrichtung des Nahtrings aufweist.

3. Anschlusseinrichtung nach Anspruch 1 oder 2, **dadurch gekennzeichnet, dass** das Dichtelement (10, 10', 29, 42) in Axialrichtung komprimierbar und dadurch in radialer Richtung aufweitbar ist.

4. Anschlusseinrichtung nach Anspruch 1 oder 2, **dadurch gekennzeichnet, dass** das Verschlusselement (7, 7') eine an seinem Umfang umlaufende Ausnehmung für das in Axialrichtung komprimierbare Dichtelement (10, 10') aufweist.

5. Anschlusseinrichtung nach Anspruch 1 oder einem der folgenden, **dadurch gekennzeichnet, dass** das Verschlusselement wenigstens zwei gesonderte und in Axialrichtung gegeneinanderpressbare Elemente (8, 8', 9, 9') aufweist, zwischen denen ein radial nach außen offener Zwischenraum, insbesondere eine am Umfang des Verschlusselements umlaufende Nut, zur Aufnahme des Dichtelementes (10, 10') gebildet ist.

6. Anschlusseinrichtung nach Anspruch 5, **dadurch gekennzeichnet, dass** am Boden der Nut wenigstens an einem der beiden gegeneinanderpressbaren Elemente eine umlaufende und radial nach außen weisende Schräge (8a, 9a) gebildet ist und dass insbesondere der Boden der Nut im Querschnitt V-förmig ausgebildet ist, wobei jeder der beiden Schenkel des V-förmigen Bodens von einem der Elemente gebildet ist.

7. Anschlusseinrichtung nach Anspruch 1 oder einem der folgenden, **dadurch gekennzeichnet, dass** das Verschlusselement zwei gegeneinanderpressbare Elemente (8, 8', 9, 9') aufweist, die durch eine zentrale Gewindeverbindung (12, 12', 13, 13') miteinander verbunden sind.

8. Anschlusseinrichtung nach Anspruch 5 oder einem der folgenden, **dadurch gekennzeichnet, dass** wenigstens ein Gewindestift (12, 12') eine Öffnung eines der gegeneinanderpressbaren Elemente (9, 9') durchsetzt und dass wenigstens eine Gewindemutter (13, 13') auf dem Gewindestift zum Gegeneinanderpressen der Elemente des Verschlusselements vorgesehen ist.

9. Anschlusseinrichtung nach Anspruch 5 oder einem der folgenden, **dadurch gekennzeichnet, dass** die beiden gegeneinanderpressbaren Elemente ineinandergreifende zentrische Gewinde aufweisen und gegeneinander verdrehbar sind.

10. Anschlusseinrichtung nach Anspruch 5 oder einem der folgenden, **dadurch gekennzeichnet, dass** die beiden gegeneinanderpressbaren Elemente eine Führungseinrichtung zur Führung ihrer Relativbewegung in Axialrichtung aufweisen.

11. Anschlusseinrichtung nach Anspruch 1 oder einem der folgenden, **dadurch gekennzeichnet, dass** das Dichtelement (10, 10', 29, 42) eine Elastomerdichtung mit insbesondere kreisrundem, ovalem, dreieckigem, viereckigem, ringförmigem, topfförmigem oder polyedrischem Querschnitt ist sowie insbesondere hohl ausgebildet ist und aus einem Schaumstoff oder Metallschaum besteht.

12. Anschlusseinrichtung nach Anspruch 1 oder einem der folgenden, **dadurch gekennzeichnet, dass** das Dichtelement ein verformbarer, insbesondere elastischer und mit einem Gas, einer Flüssigkeit oder einem Gel füllbarer Hohlkörper (10, 42'), insbesondere in Torus- oder Kugelform, ist.

13. Anschlusseinrichtung nach Anspruch 1 oder einem der folgenden, **dadurch gekennzeichnet, dass** das Verschlusselement (28, 42, 45) einen von einer elastischen Wand umgebenen Hohlraum aufweist, der zusammen mit der elastischen Wand durch Einschieben eines zapfenartigen Körpers (45) oder durch Spreizen eines Spreizelementes (30, 31, 32) innerhalb des Hohlraums radial aufweitbar ist.

14. Anschlusseinrichtung nach Anspruch 1, **dadurch gekennzeichnet, dass** das Verschlusselement auf eine Außenkontur des Nahtrings elastisch dichtend aufklemmbar ist.

15. Anschlusseinrichtung nach Anspruch 1 oder einem der folgenden, **dadurch gekennzeichnet, dass** das Verschlusselement (7, 7', 7", 7"', 7"", 25, 26, 28, 35, 39, 45) und/oder das Befestigungselement (7", 10, 10', 20, 20a, 22, 25a, 26a, 29a, 38, 42) und/oder das Dichtelement (10, 10', 20, 22, 25a, 26a, 29a, 42) jeweils ganz oder teilweise an seiner Oberfläche eine Texturierung aufweist oder mit einer texturierten Schicht, insbesondere in Form eines Textils oder Gewebes, bedeckt ist und dass insbesondere das Verschlusselement und/oder das Befestigungselement und/oder das Dichtelement aus einem Metall, einer offenporigen Metallstruktur oder einem Metallschaum, aus Titan, Kunststoff, einem offenporigen Textilgewebe oder einem organischen Schaumstoff besteht.
